Europäisches Patentamt

European Patent Office

Office européen des brevets

(11) Veröffentlichungsnummer: **0 311 898**
**B1**

# (12) EUROPÄISCHE PATENTSCHRIFT

(45) Veröffentlichungstag der Patentschrift:
03.10.90

(51) Int. Cl.⁵: **F21V 29/00**, A61N 5/06

(21) Anmeldenummer: 88116542.7

(22) Anmeldetag: 06.10.88

(54) **Bestrahlungsvorrichtung.**

(30) Priorität: 16.10.87 DE 8713930 U

(43) Veröffentlichungstag der Anmeldung:
19.04.89 Patentblatt 89/16

(45) Bekanntmachung des Hinweises auf die Patenterteilung:
03.10.90 Patentblatt 90/40

(84) Benannte Vertragsstaaten:
AT BE CH DE ES FR GB IT LI LU NL SE

(56) Entgegenhaltungen:
EP-A- 0 073 669
AU-A- 496 992
CH-A- 271 991
DE-A- 896 395
DE-U- 8 628 453
FR-A- 812 549
GB-A- 1 084 335
US-A- 4 546 420

(73) Patentinhaber: MAXS AG, Edisriederstrasse 106,
CH-6072 Sachseln(CH)

(72) Erfinder: Greutert, Albert, Sunnärai 7,
CH-6072 Sachseln(CH)

(74) Vertreter: Patentanwälte Grünecker, Kinkeldey,
Stockmair & Partner, Maximilianstrasse 58,
D-8000 München 22(DE)

## Beschreibung

Bestrahlungsvorrichtung

Die Erfindung bezieht sich auf eine Bestrahlungsvorrichtung mit einer in einem Gehäuse angeordneten Strahlenquelle und einem im Strahlengang angeordneten Filter, der zwei transparente, im wesentlichen planparallel angeordnete Scheiben aufweist, die in einem Rahmen gehalten sind und einen geschlossenen Hohlraum begrenzen, in dem ein das Strahlenspektrum selektiv beeinflussendes Medium angeordnet ist, das auch mit wenigstens einem Teil des Rahmens in Berührung steht, wobei zur Verbesserung der Wärmeabgabe Kühlrippen vorgesehen sind.

Eine solche Bestrahlungsvorrichtung ist beispielsweise aus der DE-C 896 395 bekannt. Die dort gezeigte Bestrahlungsvorrichtung dient zur Wärmetherapie des menschlichen Körpers. Zumeist ist das von der Stahlenquelle ausgesandte Strahlungsspektrum nicht in seiner Gänze für den Einsatz bei der Wärmetherapie geeignet. Daher wird der Filter vorgesetzt, um bestimmte Banden aus dem Strahlenspektrum herauszufiltern. Da der Filter im Strahlengang der Strahlenquelle angeordnet ist, ist er zugleich außerordentlich hohen Temperaturen ausgesetzt. Bei der bekannten Bestrahlungsvorrichtung wird versucht, eine zu hohe Erwärmung des in dem Filter befindlichen Mediums dadurch zu vermeiden, daß der Filter einen entsprechend großen Durchmesser aufweist und dementsprechend eine größere Wärmemenge aufnehmen kann. Da sich der Filter jedoch immer weiter aufheizt, muß nach einer bestimmten Betriebsdauer die Bestrahlungsvorrichtung abgeschaltet werden. Erfolgt ein solches Abschalten nicht, hat dies die Zerstörung des Filters zur Folge. Nachteilig an der bekannten Bestrahlungsvorrichtung ist, daß diese nur für eine bestimmte Dauer verwendet werden kann, und daß die gesamte Vorrichtung aufgrund des großen Durchmessers des Filters sehr viel Platz beansprucht. Bei dieser bekannten Bestrahlungsvorrichtung ist auch schon vorgesehen, den Filter mit Kühlrippen zur Verbesserung der Wärmeabführung auszustatten.

Die DE-C 911 525 beschreibt eine Bestrahlungsvorrichtung, bei der an dem Filter eine zusätzliche Scheibe angeordnet ist, um einen flüssigkeitsdichten Raum zu bilden, der Flüssigkeit auffängt, falls aufgrund zu hoher Temperatur eine der das Medium einschließenden Scheiben zerspringt. Das Medium, zumeist Wasser, wird dann in dem anderen Raum aufgefangen und über ein Ableitungsrohr weggeführt.

Die DE-C 7 43 499 beschreibt eine Bestrahlungsvorrichtung, bei der ein zwischen zwei Scheiben eingeschlossener Wasserfilter vorgesehen ist. In dem Rahmen ist ein Zufluß und ein Rückfluß vorgesehen, so daß durch Wasseraustausch in dem Filter die Temperatur des Filters herabgesenkt werden kann. Weiterhin beschreibt die DE-C 692 907 eine Bestrahlungsvorrichtung mit einem Wasserfilter, wobei zwischen den Scheiben des Wasserfilters diesen durchsetzende Kühlrohre angeordnet sind.

Obwohl mit dieser Art der Kühlung erreicht werden kann, daß, anders als bei der direkten Einleitung von Kühlwasser in den Wasserfilter, in dem Wasserfilter eine möglichst gleichmäßige Temperatur vorliegt, hat die bekannte Vorrichtung jedoch den Nachteil, daß die Rohre im Strahlengang der Bestrahlungsvorrichtung angeordnet sind und daher die Leistung der Vorrichtung mindern. Außerdem ist ein Vorratsbehälter für Kühlwasser und zusätzlich eine Kühlvorrichtung notwendig.

Schließlich ist noch aus dem deutschen Gebrauchsmuster DE-U 8 628 453 eine Bestrahlungsvorrichtung der eingangs genannten Art bekannt, bei der der nach Art einer Hohlraumküvette ausgebildete Wasserfilter an seiner obersten Stelle einen Auslaß aufweist, damit etwa freiwerdende Gasoder Dampfbläschen, die sich im Flüssigkeitsraum zu größeren Blasen ausbilden, entweichen können. Der Wasserfilter weist auch einen Einlaß auf, damit der Wasserfilter während des Betriebs an einen Kühlkreislauf angeschlossen werden kann.

Ein solcher Wasserkreislauf kann, insbesondere bei Verwendung von Glasscheiben nicht nur ein Zerspringen derselben verursachen, er erfordert darüber hinaus auch einen enormen apparativen Aufwand.

Schließlich ist aus der FR-A 812 549 ein Filter bekannt, der wenigstens zwei planparallele transparente Glasscheiben aufweist, wobei am Rahmen nach außen abstehende Kühlrippen angeordnet sind.

Der Erfindung liegt daher die Aufgabe zugrunde, eine Bestrahlungsvorrichtung der eingangs genannten Art in der Weise zu verbessern, daß mit möglichst geringem Aufwand eine wirksame Kühlung des Filters erreicht wird und bei Erwärmung des Filtermediums im Innern des Filters keine unzulässig hohen Druckspitzen auftreten.

Diese Aufgabe wird gemäß den Merkmalen des Anspruchs 1 gelöst.

Die Scheiben des Filters bestehen aus Kunststoff. Im Gegensatz zu Glasscheiben besitzen die Kunststoffscheiben die Möglichkeit, sich bei Erwärmen des Filtermediums, z. B. Wasser, membranartig geringfügig auszuwölben, so daß es im Inneren des Filters nicht zu unzulässigen Druckspitzen kommen kann. Besonders bewährt haben sich Scheiben aus Polycarbonat.

Aufgrund der erfindungsgemäßen Ausgestaltung führt der Rahmen, der mit dem Medium in Verbindung steht, Wärme über die Kühlrippen an die Umgebung ab. Hierdurch kann sich auch bei längerer Betriebsdauer der Bestrahlungsvorrichtung ein Gleichgewichtszustand einstellen, der es erlaubt, die Bestrahlungsvorrichtung auch über einen längeren Zeitraum zu betreiben.

Unterstützt wird die gute Kühlung des Filters, wenn der Rahmen aus Metall oder einem anderen gut wärmeleitenden Material besteht.

Ohne den guten Wärmeübergang zu beeinträchtigen, kann in vorteilhafter Weise der Rahmen zweiteilig ausgebildet sein mit einem äußeren, die Kühlrippen tragenden Rahmenteil und einem darin auswechselbar gehaltenen Wechselrahmen, an dem die Filterscheiben befestigt sind. Der Wärmeübergang ist deswegen gut, da die Trennung zwischen dem auswechselbaren Filter und dem Gehäuse am Rahmen vorgenommen wird, der aus einem gut wärmeleitenden Material bestehen kann. Sollte man die Scheiben selbst auswechselbar machen, so hätte man stets das Problem, daß darunter möglicherweise der Wärmeübergang zu dem Rahmen leiden könnte.

Der Wärmeübergang zwischen dem Filtermedium und dem Rahmen wird dadurch begünstigt, daß der Rahmen eine nach innen zwischen die Scheiben ragende, umlaufende Auflageschulter aufweist, an der die Scheiben anliegen. Damit ragt ein Bereich des gut wärmeleitenden Rahmens bis direkt an das Medium heran.

Besonders günstig ist es, wenn in der Auflageschulter eine zum Innenraum zwischen den Scheiben offene, umlaufende Kühlnut ausgebildet ist. Hierdurch wird die Auflageschulter praktisch zu zwei Kühlrippen ausgebildet, wodurch die Kontaktoberfläche zu dem Medium zwischen den beiden Scheiben wesentlich vergrößert wird. Durch diese Oberflächenvergrößerung kann soviel Wärme abgeführt werden, daß die Rahmentemperatur nur so stark ansteigt, daß in günstigen Fällen auf die äußeren Kühlrippen verzichtet werden kann. Für diese Ausführung wird daher selbständiger Schutz beansprucht.

Die zum Filtermedium weisende Oberfläche der Auflageschulter kann weiter noch dadurch vergrößert werden, daß zwei oder mehrere Kühlnuten nebeneinander vorgesehen sind, die durch einen Kühlsteg getrennt sind. Wieviel Kühlnuten insgesamt vorgesehen werden sollten, hängt nicht zuletzt von dem Abstand der beiden Scheiben zueinander ab.

Die Kühlnut ist vorzugsweise mindestens so tief wie die Auflageschulter.

Günstig ist es, wenn die Kühlnut einen V- oder U-förmigen Nutgrund aufweist. Dadurch verbreitern sich die stehengebliebenen Kühlrippen zum Rahmen hin, so daß ein gleichmäßiger Wärmefluß entstehen kann.

Im Zusammenhang mit der Kühlnut ist es günstig, wenn die Scheiben jeweils von einem Preßring gehalten sind, der die jeweilige Scheibe gegen eine Seite der Auflageschulter drückt. Dieser Preßring erlaubt eine Befestigung der Scheibe an der Auflageschulter, ohne daß diese zur Aufnahme einer Befestigungsschraube durchbohrt werden muß.

Ohne den Wärmeübergang zu beeinträchtigen, läßt sich der das Filtermedium aufweisende Dichtraum besonders einfach dadurch abdichten, daß die Scheiben an ihrem, der Auflageschulter zugewandten Außenrand eine Schräge aufweisen, und daß zwischen der Schräge und der Auflageschulter ein Dichtring angeordnet ist.

Auf eine Befestigung der Scheiben des Filters an dem Rahmen mittels Schrauben kann in vorteilhafter Weise verzichtet werden, wenn der der jeweiligen Scheibe abgewandte äußere Rand eines Preßringes leicht angeschrägt ist, und wenn in der äußeren Stirnseite des die Preßringe umgebenden Rahmens eine umlaufende, einen Bördelsteg stehenlassende Nut vorgesehen ist. Nach Einsetzen des Preßringes braucht dieser Bördelsteg lediglich nach innen eingerollt zu werden, wo er dann die Schräge des Preßringes hintergreift und diesen gegen die Scheibe gedrückt hält. Hierdurch wird eine innige Berührung zwischen dem Preßring und dem Rahmen erreicht, was der Wärmeleitung und damit der Wärmeabfuhr aus dem Filter zugutekommt.

Eine andere vorteilhafte Möglichkeit, die Wärme besser aus dem Medium abführen, ergibt sich, wenn an dem Rahmen nach innen zwischen die Scheiben in das Medium tauchende Lamellen angebracht sind.

Will man auch bei kompakter Bauweise der Bestrahlungsvorrichtung eine gute Kühlung des Filters erreichen, so ist es günstig, wenn das Gehäuse im wesentlichen rohrförmig ausgebildet ist und der Rahmen mit den äußeren Enden seiner Kühlrippen unter Freilassung von Luftströmungsöffnungen in das Gehäuse eingesetzt ist. Das Gehäuse wirkt, wenn es senkrecht steht, als eine Art Kamin und unterstützt eine Umströmung der Kühlrippen mit kühler Luft.

Eine gute Umströmung der Kühlrippen kann in baulich einfacher Weise auch dadurch erzielt werden, daß in dem Gehäuse ein Ventilator angeordnet ist. Hierdurch kann die Bestrahlungsvorrichtung auch bei kompakter Bauweise in jeder beliebigen Lage verwendet werden, ohne daß der Filter überhitzt wird.

Gemäß einer vorteilhaften Weiterbildung der Erfindung ist der Ventilator auf der dem Filter abgewandten Seite der Strahlenquelle angeordnet. Der Ventilator saugt so zunächst Frischluft über die Kühlrippen des Filters in das rohrförmige Gehäuse, wobei die bereits geringfügig aufgeheizte Luft auch zu einer Abkühlung der Strahlenquelle beiträgt.

Insbesondere dann, wenn das Medium in dem Filter einschließenden Scheiben aus einem Kunststoff bestehen, ist es vorteilhaft, wenn zwischen der der Strahlenquelle zugewandten Scheibe des Filters und der Strahlenquelle eine ringartige Strömungsschikane konzentrisch zum Gehäuse angeordnet ist, deren Strömungseinlauf den Kühlrippen des Rahmens zugewandt ist, während deren Strömungsauslauf zum Filter weist. Auf diese Weise wird die in das Gehäuse eintretende Luft nach dem Passieren der Kühlrippen auf die der Strahlenquelle zugewandte Scheibe gelenkt, wodurch die betreffende Scheibe in vorteilhafter Weise gekühlt wird.

Eine besonders weiche Umlenkung der Kühlluft und damit eine wirksame Kühlung bei geringem Strömungsverlust, läßt sich dadurch erreichen, daß die Strömungsschikane als Torushälfte ausgebildet ist, deren äußerer Rand an die Innenwand des rohrförmigen Gehäuses angrenzt und deren innerer Rand zum Filter weist.

Eine baulich besonders einfache Strömungsschikane läßt sich dadurch erreichen, daß die Kühlrippen gehäuseeinwärts verlängert sind und daß die Strömungsschikane als die Fußbereiche der Kühlrippen verbindender zylindrischer Ring ausgebildet ist, der gegenüber dem Gehäuse durch einen Flanschteil abgeschlossen ist und der von der inneren Scheibe des Filters zur Bildung des Strömungsauslaufs beabstandet angeordnet ist. Das hat den Vorteil, daß die Strömungsschikane aus einem Guß mit dem Rahmen und den Kühlrippen hergestellt werden kann. Die verlängerten Kühlrippen bewirken darüber hinaus eine noch bessere Wärmeableitung.

Günstig ist auch, wenn konzentrisch zu dem Gehäuse zwischen der Strahlenquelle und dem Filter eine hitzebeständige Glasplatte angeordnet ist, deren Rand durch einen im wesentlichen geschlossenen Ringspalt zur Innenwand des Gehäuses beabstandet ist. Dieser Glasplatte kommt eine Doppelfunktion zu. Zum einen schirmt sie den Filter bereits vor einer übermäßigen Wärmeentwicklung ab. Zum anderen bildet sie eine weitere Strömungsschikane für die über die Kühlrippen und die erste Strömungsschikane eintretende Luft. Die eintretende Luft streicht hierdurch besonders lange an der der Strahlenquelle zugewandten Scheibe des Filters entlang.

Verstärkt werden kann dieser Effekt dadurch, daß der Innendurchmesser des Ringspaltes größer ist als der Durchmesser des inneren Randes der Strömungsschikane. Hierdurch erfolgt eine Umlenkung der entlang der inneren Scheibe radial nach innen strömenden Luft um 180°, so daß diese Luft anschließend radial nach außen strömen muß. Hierdurch findet eine gute Durchmischung der Kühlluft statt, was den Wärmeaustausch und damit die Wärmeabfuhr durch die Kühlluft begünstigt.

Gemäß einer einfachen baulichen Ausgestaltung der Erfindung ist die Strahlenquelle als Halogenlampe mit zum Filter weisendem Reflektor ausgebildet.

Im folgenden werden Ausführungsbeispiele der Erfindung anhand einer Zeichnung näher erläutert. Es zeigen:

Fig. 1 in einem schematisch dargestellten Längsschnitt eine erste Ausführungsform einer erfindungsgemäßen Bestrahlungsvorrichtung,

Fig. 2 eine zweite Ausführungsform einer erfindungsgemäßen Bestrahlungsvorrichtung in ähnlicher Ansicht wie in Fig. 1,

Fig. 3 eine Ansicht auf die Bestrahlungsvorrichtung aus Fig. 1 in Richtung des Pfeiles III,

Fig. 4 den vorderen Teil einer Bestrahlungsvorrichtung gemäß einer dritten Ausführungsform, teilweise im Längsschnitt,

Fig. 5 eine Ansicht auf die Bestrahlungsvorrichtung aus Fig. 4 in Richtung des Pfeiles V,

Fig. 6 eine Detailansicht VI aus Fig. 4 gemäß einer ersten Variante,

Fig. 7 in ähnlicher Ansicht wie Fig. 6 das Detail gemäß einer zweiten Variante,

Fig. 8 in ähnlicher Ansicht wie Fig. 6 das Detail gemäß einer dritten Variante, und Fig. 9a,

Fig. 9b das Detail IX aus Fig. 4, einmal vor und einmal nach einer Verbördelung.

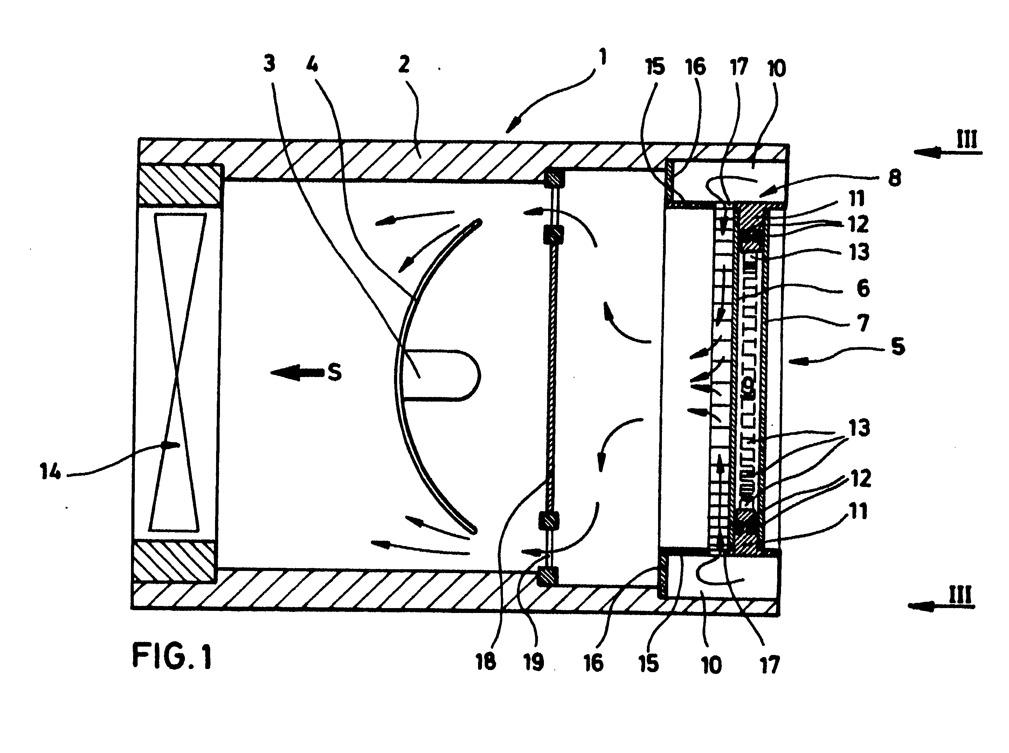

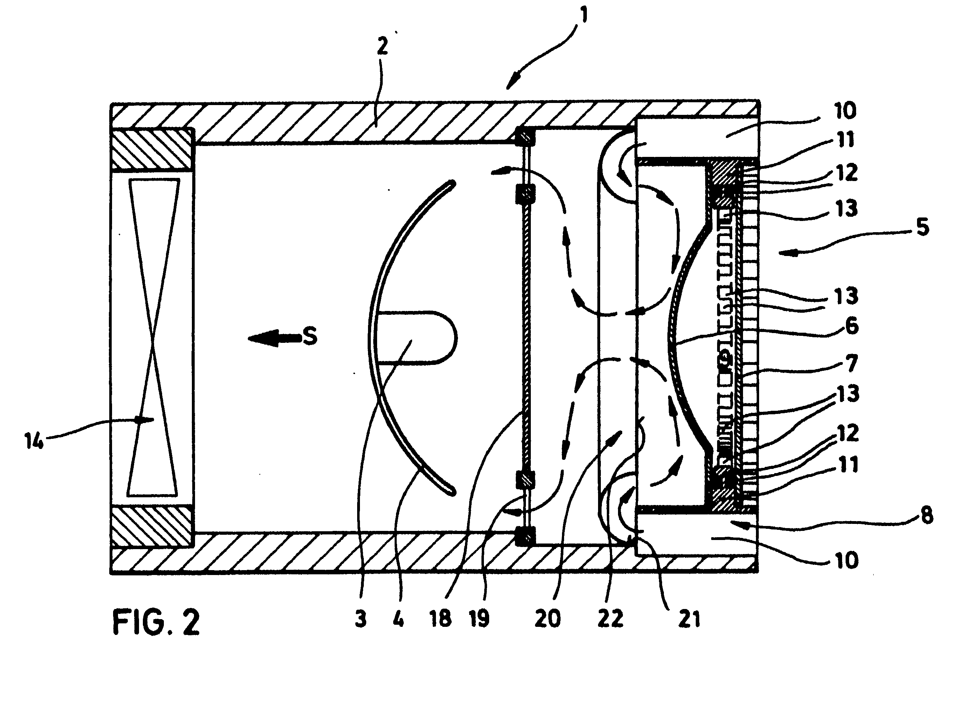

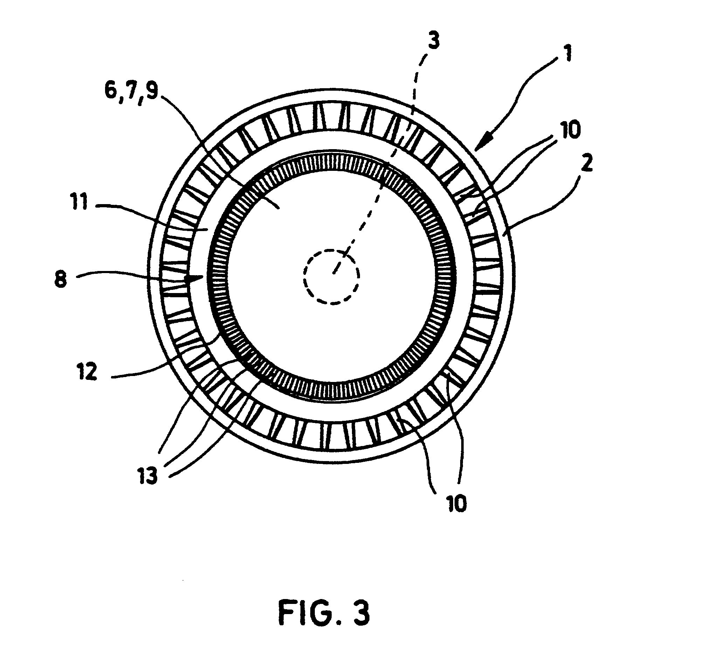

Die Figuren 1 und 3 zeigen eine Bestrahlungsvorrichtung 1 mit in einem Gehäuse 2 angeordneter Strahlenquelle 3 in Form einer Halogenlampe, hinter der ein Reflektor 4 angeordnet ist. Im Strahlengang der Halogenlampe 3 ist ein Filter 5 angeordnet. Der Filter 5 besteht aus zwei planparallel angeordneten Kunststoffscheiben 6 und 7 aus Polycarbonat. Die beiden Kunststoffscheiben 6 und 7 sind durch einen Rahmen 8 beabstandet voneinander gehalten und schließen einen Hohlraum 9 ein, der mit Wasser gefüllt ist. Das Wasser in dem Hohlraum 9 beeinflußt das Strahlenspektrum der von der Halogenlampe 3 ausgesendeten Strahlen, in dem es in Zusammenwirken mit den beiden Polycarbonatscheiben 6 und 7 Strahlen der Wellenlänge 1300 bis 1600 nm herausfiltert. Es handelt sich hierbei um Wellenlängen, die vom Körpergewebe stark absorbiert werden und daher für die Wärmetherapie weniger geeignet sind.

Der Rahmen 8 weist an seinem Außenumfang nach außen abstehende Kühlrippen 10 auf, die einstückig mit dem übrigen Rahmen 8 verbunden sind.

Von dem Außenumfang des Rahmens 8 aus erstreckt sich ein Steg 11 radial nach innen, an die beiden Scheiben 6 und 7 anliegen. Auf beiden Seiten des Steges 11 sind Axialnuten vorgesehen, in denen ein Dichtring 12 eingelegt ist, der die beiden Scheiben 6 und 7 gegenüber dem Steg 11 abdichtet. Von dem Steg 11 aus erstrecken sich weiter radial nach innen gerichtete Lamellen 13, die in das in dem Hohlraum 9 befindliche Wasser eintauchen.

Das Gehäuse 2 ist im wesentlichen als Hohlzylinder ausgebildet, wobei der Filter 5 mit den äußeren Enden der Kühlrippen 10 seines Rahmens 8 in das Gehäuse 2 eingesetzt ist. Genau genommen befindet sich der Filter 5 an einem stirnseitigen Ende des Gehäuses 2.

Auf der dem Filter 5 abgewandten Seite der Halogenlampe 3 ist ein Ventilator 14 angeordnet, der, wenn er in Betrieb ist, Luft in Pfeilrichtung S ansaugt.

Der Rahmen 8 ist im Bereich der Kühlrippen 10 so ausgebildet, daß er eine Strömungsschikane bildet. Hierzu ist an der Unterseite der ins Gehäuseinnere ragenden Kühlrippen 10 ein zylinderartiger Ring 15 befestigt, dessen ins Gehäuseinnere weisende Stirnseite durch ein Flanschteil 16 gegenüber dem Gehäuse 2 abgedichtet ist. Zwischen der Scheibe 6 des Filters 5 und dem Ring 15 verbleibt ein Spalt 17, der lediglich durch die übergreifenden Kühlrippen 10 unterbrochen ist. Aus dieser Anordnung ergibt sich, daß die aus dem Ring 15 und dem Flanschteil 16 gebildete Strömungsschikane einen den Kühlrippen 10 zugewandten Strömungseinlauf und einen der Scheibe 6 zugewandten Strömungsauslauf in Form des Spaltes 17 aufweist.

Zwischen der Halogenlampe 3 und dem Filter 5 ist eine hitzebeständige Glasplatte 18 angeordnet, deren Rand durch einen im wesentlichen geschlossenen Ringspalt 19 zur Innenwand des Gehäuses 2 beabstandet ist. Der Ringspalt 19 wird lediglich durch Halterungen für die Glasplatte 18 unterbrochen.

Aus Fig. 1 ist deutlich zu erkennen, daß die Dicke des Filters 5 etwa einem Drittel der Länge der Kühlrippen 10 entspricht und daß die Kühlrippen 10 in Richtung des Gehäuseinneren verlängert ausgebildet sind.

Im folgenden wird die Wirkungsweise des Ausführungsbeispieles gemäß den Fig. 1 und 3 näher erläutert.

Zum Betrieb der Bestrahlungsvorrichtung 1 werden zunächst die Halogenlampe 3 und der Ventilator 14 angeschaltet. Die Halogenlampe sendet, gebündelt durch den Reflektor 4, Strahlen in Richtung des Filters 5 aus. Diese Strahlen treffen zunächst auf die hitzebeständige Glasplatte 18 und dann auf den Filter 5 auf. Hierdurch erwärmen sich die Kunststoffscheiben 6 und 7 sowie das in dem Hohlraum 9 befindliche Wasser, obgleich die hitzebeständige Glasplatte einen Teil der Wärme zurückhält. Das im Hohlraum 9 erwärmte Wasser gibt die Wärme über die in das Wasser eintauchenden Lamellen 13 an den aus Aluminium bestehenden Rahmen 8 und dessen Kühlrippen 10 ab.

Da der Ventilator läuft, wird entlang der Kühlrippen 10 Luft angesaugt, die in Pfeilrichtung ringsum den Filter radial nach innen entlang der inneren Kunststoffscheibe 6 strömt und dabei die Scheibe 6 kühlt. Die Luft gelangt von dort durch den Ringspalt 19 zum Ventilator 14 und von dort ins Freie.

Im folgenden wird das Ausführungsbeispiel gemäß Fig. 2 näher erläutert. Der Aufbau der in Fig. 2 gezeigten Bestrahlungsvorrichtung ist prinzipiell derselbe, wie bei dem in Fig. 1 beschriebenen Ausführungsbeispiel.

Es werden daher für gleichwirkende Bauteile identische Bezugzeichen verwendet. Es erfolgt ebenfalls lediglich eine Beschreibung der Unterschiede zur Bestrahlungsvorrichtung gemäß Fig. 1.

Die innere Kunststoffscheibe 6 ist beim Ausführungsbeispiel gemäß Fig. 2 konvex gewölbt ausgebildet, wodurch eine weitere Bündelung der von der Halogenlampe 3 ausgesendeten Strahlen erreicht werden kann.

Die Strömungsschikane ist beim zweiten Ausführungsbeispiel als Torushälfte 20 ausgebildet, deren äußerer Rand 21 an die Innenwand des Gehäuses 2 angrenzt. Der innere Rand 22 der Torushälfte 20 weist zur Scheibe 6.

Es ist zu erkennen, daß die Torushälfte 20 sich direkt an die Kühlrippen 10 des Rahmens 8 anschließt. Der Durchmesser des inneren Randes 22 der Torushälfte 20 ist zudem kleiner als der Innendurchmesser des Ringspaltes 19 ausgebildet.

Die Wirkungsweise der Bestrahlungsvorrichtung gemäß dem zweiten Ausführungsbeispiel ist prinzipiell dieselbe wie die der bereits oben beschriebenen Bestrahlungsvorrichtung. Eine Besonderheit liegt jedoch darin, daß aufgrund der sanften Umleitung der Luftströmung durch die Torushälfte 20 und die konvexe Wölbung der Scheibe 6 in Verbindung mit den Durchmesserunterschieden zwischen dem inneren Rand 22 der Torushälfte 20 und dem Innendurchmesser des Ringspaltes 19 eine besoders gute Ausnutzung der Kühlluft, insbesondere zur Kühlung der Scheibe 6 hervorgerufen wird.

Es bleibt noch anzumerken, daß die durch den Ventilator 14 hervorgerufene Luftströmung auch eine Kühlung der Halogenlampe 3 bewirkt.

Die Kunststoffscheiben 6 und 7 bestehen aus Polycarbonat und können auch eingefärbt sein, um bestimmte andere Wellenlängen aus dem Strahlenspektrum herauszufiltern. Ebenso ist es möglich, anstelle von Wasser eine Salzlösung als Medium zu verwenden. Anstatt einer konvexen Scheibe können auch zwei konvexe Scheiben vorgesehen sein. Die Lamellen können auch in Form einer einzigen Ringlamelle ausgebildet sein. Es ist möglich, das Wasser in dem Hohlraum 9 zu färben oder mit anderen das Strahlenspektrum beeinflussenden Substanzen zu versetzen.

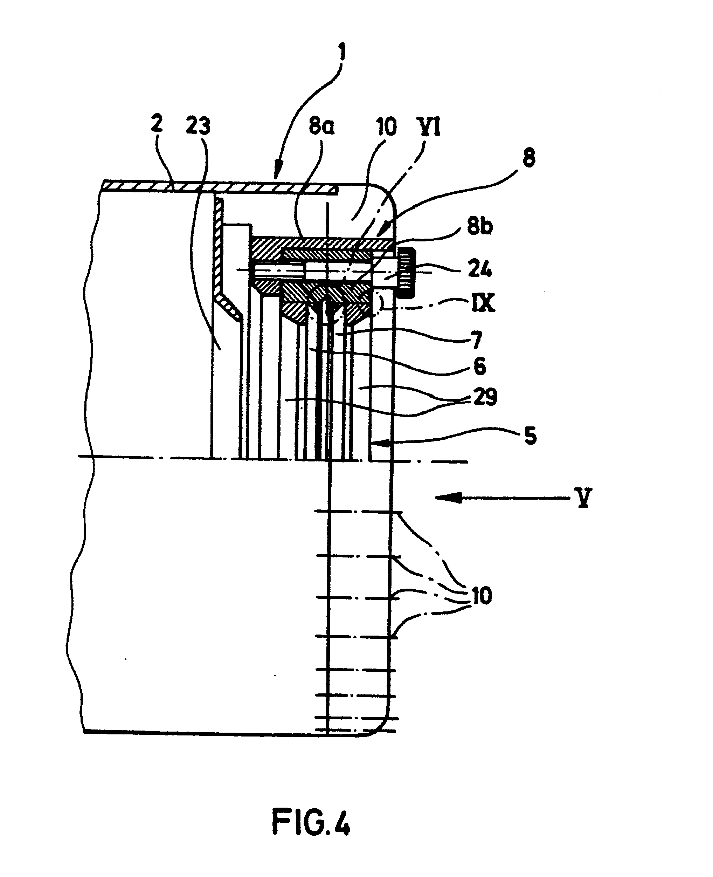

In den Fig. 4 bis 9 ist ein drittes Ausführungsbeispiel einer erfindungsgemäßen Bestrahlungsvorrichtung 1 dargestellt.

Das dritte Ausführungsbeispiel stimmt im wesentlichen mit den beiden zuvor beschriebenen Ausführungsbeispielen überein, weshalb für gleiche und ähnliche Bauteile dieselben Bezugzeichen verwendet werden. Im folgenden wird nur auf die wesentlichen Unterschiede eingegangen.

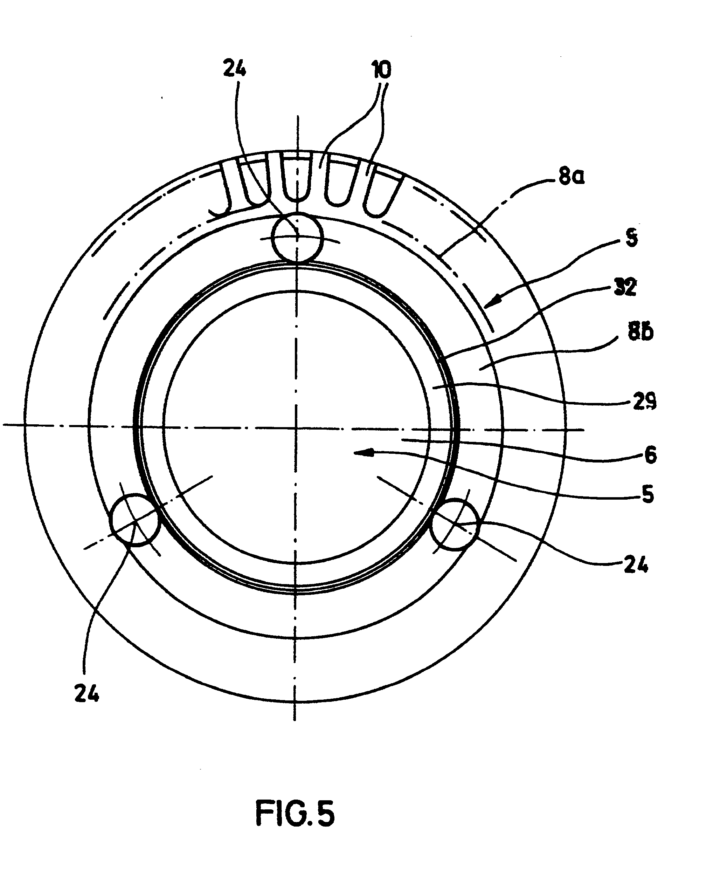

Wie deutlich aus Fig. 4 und 5 ersichtlich ist, ist der Filter 5 als Wechselfilter ausgebildet, in dem der Rahmen 8 aus zwei Rahmenhälften 8a und 8b besteht. Der äußere Rahmenteil 8a trägt die Kühlrippen 10 mit denen er mit dem Gehäuse 2 verbunden ist. Am inneren Ende der Kühlrippen 10 ist ein Luftleitblech 23, ähnlich der Torushälfte 22 angeordnet. Der äußere Rahmenteil 8a ist mit einer konzentrischen Ausnehmung versehen, in welche der innere Rahmenteil, der Wechselrahmen 8b eingesetzt ist. Der Wechselrahmen 8b kann über Rändelschrauben 24 an dem äußeren Rahmen 8a angeschraubt werden.

An dem Wechselrahmen 8b ist eine radial nach innen ragende Auflageschulter vorgesehen, an deren Außenseiten die beiden Scheiben 6 und 7 anliegen. In der Auflageschulter ist eine umlaufende, radial nach innen offene Nut 26 ausgebildet.

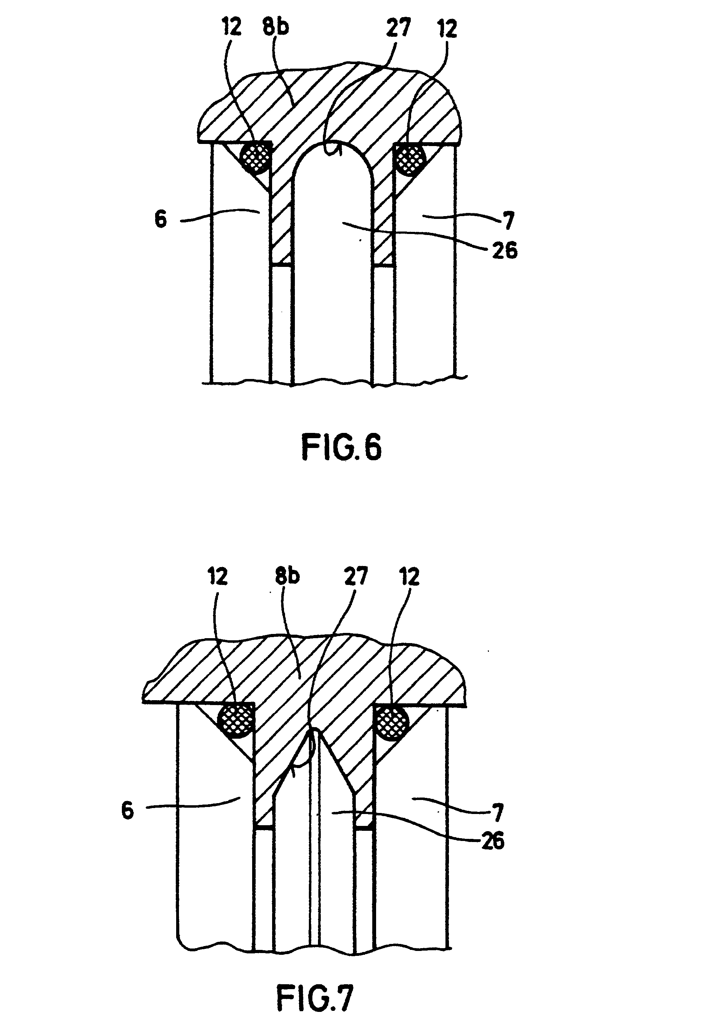

In den Fig. 6 bis 8 sind drei Varianten der Auflageschulter näher dargestellt.

In der in Fig. 6 dargestellten, ersten Variante der Aufl-geschulter ist nur eine Nut 26 vorgesehen, die genauso tief ist, wie die Auflageschulter selbst. Der Nutgrund 26 ist U-förmig ausgebildet, so daß sich die stehengebliebenen Rippen zum Wechselrahmen 8b hin erweitern.

Bei der in Fig. 7 dargestellten Variante ist der Nutgrund 27 der Nut 26 V-förmig ausgebildet. Auch hier verbreitern sich die stehengebliebenen Rippen radial nach außen zum Wechselrahmen 8b.

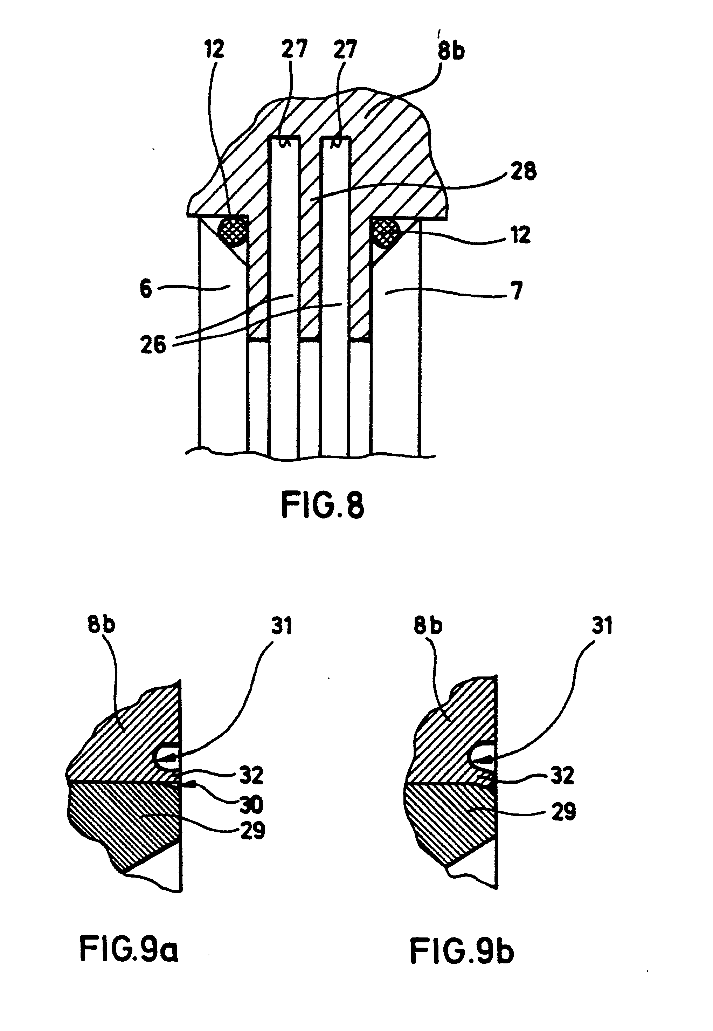

Bei der in Fig. 8 dargestellten dritten Variante sind nebeneinander zwei Kühlnuten 26 vorgesehen, die tiefer sind als die Auflageschulter. Zwischen den beiden Nuten 26 verbleibt ein Kühlsteg 28.

Wie aus den Fig. 6 bis 8 weiterhin gut ersichtlich ist, weisen die Scheiben 6 und 7 an ihrem äußeren Rand eine den Stirnseiten der Auflageschulter zugewandte Schräge auf. Hierdurch wird zwischen der Auflageschulter und der Scheibe ein im Querschnitt dreieckförmiger Raum gebildet. In diesem Raum ist jeweils ein Dichtring 12 eingelegt.

Die Befestigung der Scheiben 6 und 7 erfolgt bei dem dritten Ausführungsbeispiel durch zwei Preßringe 29, die die Scheiben 6 bzs. 7 gegen die Stirnseiten der Auflageschulter drücken. Die Preßringe 29 weisen an ihrem, den Scheiben 6, 7 abgewandten äußeren Rand eine leichte Schräge 30 auf. An der Stirnseite des Wechselrahmens 8b, der die Preßringe 29 außen umgibt, ist auf beiden Seiten eine Umlaufnut 31 vorgesehen und zwar mit einem radialen Abstand zum Preßring, daß am radial inneren Rand des Wechselrahmens 8b ein Bördelsteg 32 verbleibt. Im Detail ist diese Anordnung in den Fig. 9a und 9b zu sehen. In der Fig. 9a ist der Preßring 29 zwar eingesetzt, jedoch noch nicht verbördelt. Die Fig. 9b zeigt den umgebördelten Bördelsteg 32, wie er die Schräge 30 des Preßringes 29 hintergreift.

Im folgenden wird die Wirkungsweise des dritten Ausführungsbeispieles näher erläutert.

Der Betrieb der Bestrahlungsvorrichtung erfolgt in gleicher Weise wie bei den zuvor beschriebenen Ausführungsbeispielen. Das zwischen den Scheiben 6 und 7 befindliche Wasser wird gekühlt, indem die Wärme über die durch die Kühlnuten 26 vergrößerte Oberfläche der Auflageschulter in den Wechselrahmen 8b abgeführt wird. Von dem Wechselrahmen 8b gelangt die Wärme dann über eine verhältnismäßig große Kontaktfläche zu dem äußeren Rahmenteil 8a und von dort zu den Kühlrippen. Der Filter 5 kann auf einfache Weise durch Lösen der Rändelschrauben 24 ausgewechselt werden. Wegen der großen Kontaktfläche zwischen dem Außenrahmen 8a und dem Wechselrahmen 8b bewirkt die Auswechselbarkeit keine Beeinträchtigung der guten Wärmeabfuhr aus dem Filter 5.

Obwohl die Bestrahlungsvorrichtung bei den Ausführungsbeispielen anhand eines zylinderförmigen Gehäuses und demzufolge auch eines kreisförmigen Filters beschrieben wurde, ist es denkbar, den Filter rechteckig oder in Form eines anderen Vielecks auszubilden.

## Patentansprüche

1. Bestrahlungsvorrichtung mit einer in einem Gehäuse (2) angeordneten Strahlenquelle (3) und einem im Strahlengang angeordneten Filter (5), der zwei transparente, im wesentlichen planparallel angebrachte Scheiben (6, 7), aufweist, die in einem Rahmen (8) gehalten sind und einen geschlossenen Hohlraum begrenzen, in dem ein das Srahlenspektrum selektiv beeinflussendes Medium angeordnet ist, das auch mit wenigstens einem Teil des Rahmens (8) in Berührung steht, wobei zur Verbesserung der Wärmeabgabe Kühlrippen (10) vorgesehen sind, dadurch gekennzeichnet, daß die Kühlrippen (10) am Außenumfang des Rahmens (8) angeordnet sind und als nach außen abstehend, bis an eine Innenwandung des Gehäuses (2) reichend ausgebildet sind, und daß mindestens eine der Scheiben (6, 7) aus einer sich bei Druckanstieg im Hohlraum membranartig nach außen wölbenden Kunststoffscheibe besteht.

2. Bestrahlungsvorrichtung nach Anspruch 1, dadurch gekennzeichnet, daß der Rahmen (8) aus Metall oder einem anderen gut wärmeleitbaren Material besteht.

3. Bestrahlunsvorrichtung nach Anspruch 1 oder 2, dadurch gekennzeichnet, daß der Rahmen (8) zweiteilig ausgebildet ist mit einem äußeren, die Kühlrippen (10) tragenden Rahmenteil (8a) und einem darin auswechselbar gehaltenen Wechselrahmen (8b) an dem die Scheiben (6, 7) befestigt sind.

4. Bestrahlungsvorrichtung nach einem der Ansprüche 1 bis 3, dadurch gekennzeichnet, daß der Rahmen (8) eine nach innen zwischen die Scheiben (6, 7) ragende, umlaufende Auflageschulter aufweist, an der die Scheiben (6, 7) anliegen.

5. Bestrahlungsvorrichtung nach einem der Ansprüche 1 bis 4, dadurch gekennzeichnet, daß in der Auflageschulter mindestens eine, zum Innenraum zwischen den Scheiben (6, 7) offene, umlaufende Kühlnut (26) ausgebildet ist.

6. Bestrahlungsvorrichtung nach einem der Ansprüche 1 bis 5, dadurch gekennzeichnet, daß in der Auflageschulter zwei Kühlnuten (26) nebeneinander vorgesehen sind, die durch einen Kühlsteg (28) getrennt sind.

7. Bestrahlungsvorrichtung nach einem der Ansprüche 1 bis 6, dadurch gekennzeichnet, daß die Kühlnut (26) mindestens so tief ist wie die Auflageschulter.

8. Bestrahlungsvorrichtung nach einem der Ansprüche 1 bis 7, dadurch gekennzeichnet, daß die Kühlnut (26) einen V- oder U-förmigen Nutgrund (27) aufweist.

9. Bestrahlungsvorrichtung nach einem der Ansprüche 1 bis 8, dadurch gekennzeichnet, daß die Scheiben (6, 7) jeweils von einem Preßring (29) gehalten sind, der die jeweilige Scheibe (6 bzw. 7) gegen eine Seite der Auflageschulter drückt.

10. Bestrahlungsvorrichtung nach einem der Ansprüche 1 bis 9, dadurch gekennzeichnet, daß die Scheiben (6, 7) an ihrem der Auflageschulter zugewandten Außenrand eine Schräge aufweisen, und daß zwischen der Schräge und der Auflageschulter ein Dichtring (12) angeordnet ist.

11. Bestrahlungsvorrichtung nach einem der Ansprüche 1 bis 10, dadurch gekennzeichnet, daß der der jeweiligen Scheibe (6, 7) abgewandte äußere Rand eines Preßringes (29) leicht angeschrägt ist, und daß in der äußeren Stirnseite des die Preßringe (29) umgebenden Rahmens (8b) eine umlaufende, einen Bördelsteg (32) stehenlassende Nut (31) vorgesehen ist.

12. Bestrahlungsvorrichtung nach einem der Ansprüche 1 bis 11, dadurch gekennzeichnet, daß beide Scheiben (6, 7) aus Kunststoff bestehen.

13. Bestrahlungsvorrichtung nach einem der Ansprüche 1 bis 12, dadurch gekennzeichnet, daß die Scheiben (6, 7) aus Polycarbonat bestehen.

14. Bestrahlungsvorrichtung nach einem der Ansprüche 1 bis 13, dadurch gekennzeichnet, daß an dem Rahmen (8) nach innen zwischen die Scheiben (6, 7) in das Medium tauchende Lamellen (13) angebracht sind.

15. Bestrahlungsvorrichtung nach einem der Ansprüche 1 bis 14, dadurch gekennzeichnet, daß das Gehäuse (2) im wesentlichen rohrförmig ausgebildet ist und daß der Rahmen (8) mit den äußeren Enden seiner Kühlrippen (10) unter Freilassung von Luftströmungsöffnungen in das Gehäuse (2) eingesetzt ·ist.

16. Bestrahlungsvorrichtung nach einem der Ansprüche 1 bis 15, dadurch gekennzeichnet, daß in dem Gehäuse (2) ein Ventilator (14) angeordnet ist.

17. Bestrahlungsvorrichtung nach einem der Ansprüche 1 bis 16, dadurch gekennzeichnet, daß der Ventilator (14) auf der dem Filter (5) abgewandten Seite der Strahlenquelle (3) angeordnet ist.

18. Bestrahlungsvorrichtung nach einem der Ansprüche 1 bis 17, dadurch gekennzeichnet, daß zwischen der der Strahlenquelle (3) zugewandten Scheibe (6) des Filters (5) und der Strahlenquelle (3) eine ringartige Strömungsschikane (15, 16; 20) konzentrisch zum Gehäuse (2) angeordnet ist, deren Strömungseinlauf den Kühlrippen (10) des Rahmens (8) zugewandt ist, während deren Strömungsauslauf (17) zum Filter (5) weist.

19. Bestrahlungsvorrichtung nach einem der Ansprüche 1 bis 18, dadurch gekennzeichnet, daß die Strömungsschikane als Torushälfte (20) ausgebildet ist, deren äußerer Rand (21) an die Innenwand des rohrförmigen Gehäuses (2) angrenzt und deren innerer Rand (22) zum Filter (5) weist.

20. Bestrahlungsvorrichtung nach einem der Ansprüche 1 bis 18, dadurch gekennzeichnet, daß die Kühlrippen (10) gehäuseeinwärts verlängert sind und daß die Strömungsschikane als die Fußbereiche der Kühlrippen (10) verbindender zylindrischer Ring (15) ausgebildet ist, der gegenüber dem Gehäuse (2) durch einen Flanschteil (10) abgeschlossen ist und der von der inneren Scheibe (6) des Filters (5) zur Bildung des Strömungslaufs beabstandet angeordnet ist.

21. Bestrahlungsvorrichtung nach einem der Ansprüche 1 bis 20, dadurch gekennzeichnet, daß konzentrisch zu dem Gehäuse (2) zwischen der Strahlenquelle (3) und dem Filter (5) eine hitzebeständige Glasplatte (18) angeordnet ist, deren Rand durch einen im wesentlichen geschlossenen Ringspalt (19) zur Innenwand des Gehäuses (2) beabstandet ist.

22. Bestrahlungsvorrichtung nach einem der Ansprüche 1 bis 21, dadurch gekennzeichnet, daß der Innendurchmesser des Ringspaltes (19) größer ist als der Durchmesser des inneren Randes (22) der Strömungsschikane (20).

23. Bestrahlungsvorrichtung nach einem der Ansprüche 1 bis 22, dadurch gekennzeichnet, daß die Strahlenquelle (3) als Halogenlampe mit zum Filter (5) weisendem Reflektor (4) ausgebildet ist.

24. Filter (5), der zwei transparente, im wesentlichen planparallel angebrachte Scheiben (6, 7) aufweist, die in einem Rahmen (8) gehalten sind und einen geschlossenen Hohlraum begrenzen, in dem ein das Strahlenspektrum selektiv beeinflussendes Medium angeordnet ist, das auch mit wenigstens einem Teil des Rahmens (18) in Berührung steht, wobei zur Verbesserung der Wärmeabgabe am Außenumfang des Rahmens (8) ·Kühlrippen (10) vorgesehen sind, dadurch gekennzeichnet, daß die nach außen abstehenden Kühlrippen (10) bis an eine Innenwandung eines Gehäuses (2) reichen, und daß mindestens eine der Scheiben (6, 7) aus einer sich bei Druckanstieg im Hohlraum membranartig nach außen wölbenden Kunststoffscheibe besteht.

25. Filter nach Anspruch 24, der nach dem kennzeichnenden Teil einer der Ansprüche 2 bis 14 weitergebildet ist.

## Claims

1. Radiotherapy device having a radiation source (3) arranged in a housing (2) and a filter (5), which is arranged in the beam path and has two transparent discs (6, 7), which are fitted essentially in a plane-parallel fashion, are held in a frame (8), and delimit a closed cavity in which a medium that selectively influences the radiation spectrum is arranged, and which medium is also in contact with at least a part of the frame (8), cooling ribs (10) being provided to improve the dissipation of heat, characterized in that the cooling ribs (10) are arranged on theouter circumference of the frame (8) and constructed to project outwards and reach as far as an inner wall of the housing (2), and in that at least one of the discs (6, 7) consists of a plastic disc that bends outwards like a diaphragm given a pressure rise in the cavity.

2. Radiotherapy device according to Claim 1, characterized in that the frame (8) consists of metal or of another material that is a good conductor of heat.

3. Radiotherapy device according to Claim 1 or 2, characterized in that the frame (8) is constructed in two parts, having an outer frame part (8a), which bears the cooling ribs (10), and an exchange frame (8b), held replaceably therein, to which the discs (6, 7) are fastened.

4. Radiotherapy device according to one of Claims 1 to 3, characterized in that the frame (8) has a circumferential seating shoulder, which projects inwards between the discs (6, 7) and against which the discs (6, 7) bear.

5. Radiotherapy device according to one of Claims 1 to 4, characterized in that at least one circumferential cooling groove (26) that is open towards the interior between the discs (6, 7) is constructed in the seating shoulder.

6. Radiotherapy device according to one of Claims 1 to 5, characterized in that two cooling grooves (26) that are separated by a cooling web (28) are provided next to one another in the seating shoulder.

7. Radiotherapy device according to one of Claims 1 to 6, characterized in that the cooling groove (26) is at least as deep as the seating shoulder.

8. Radiotherapy device according to one of Claims 1 to 7, characterized in that the cooling groove (26) has a V-shaped or U-shaped groove face (27).

9. Radiotherapy device according to one of Claims 1 to 8, characterized in that the discs (6, 7) are each held by a clamping ring (29), which presses the respective disc (6 or 7) against a side of the seating shoulder.

10. Radiotherapy device according to one of Claims 1 to 9, characterized in that the discs (6, 7) have a bevel on their outer edge that faces the seating shoulder, and in that a sealing ring (12) is arranged between the bevel and the seating shoulder.

11. Radiotherapy device according to one of Claims 1 to 10, characterized in that the outer edge of one clamping ring (29), which edge is averted from the respective disc (6, 7), is slightly bevelled, and in that a circumferential groove (31) that leaves a flanged web (32) standing is provided in the outer end face of the frame (8b) surrounding the clamping rings (29).

12. Radiotherapy device according to one of Claims 1 to 11, characterized in that both discs (6, 7) consist of plastic.

13. Radiotherapy device according to one of Claims 1 to 12, characterized in that the discs (6, 7) consist of polycarbonate.

14. Radiotherapy device according to one of Claims 1 to 13, characterized in that fins (13) dipping inwards into the medium between the discs (6, 7) are fitted to the frame (8).

15. Radiotherapy device according to one of Claims 1 t 14, characterized in that the housing (2) is of an essentially tubular construction, and in that the frame (8) is inserted with the outer ends of its cooling ribs (10) into the housing (2), leaving air-flow openings free.

16. Radiotherapy device according to one of Claims 1 to 15, characterized in that a ventilator (14) is arranged in the housing (2).

17. Radiotherapy device according to one of Claims 1 to 16, characterized in that the ventilator (14) is arranged on the side of the radiation source (3) which is averted from the filter (5).

18. Radiotherapy device according to one of Claims 1 to 17, characterized in that there is arranged between the disc (6) of the filter (5), which disc faces the radiation source (3), and the radiation source (3) an annular flow baffle (15, 16; 20), which is concentric with the housing (2) and whose flow inlet faces the cooling ribs (10) of the frame (8), while its flow outlet (17) points towards the filter (5).

19. Radiotherapy device according to one of Claims 1 to 18, characterized in that the flow baffle is constructed as half a torus (20) of which the outer edge (21) adjoins the inner wall of the tubular housing (2), and of which the inner edge (22) points towards the filter (5).

20. Radiotherapy device according to one of Claims 1 to 18, characterized in that the cooling ribs (10) are extended inwards of the housing, and in that the flow baffle is constructed as a cylindrical ring (15), which connects the base regions of the cooling ribs (10), is sealed with respect to the housing (2) by a flanged part (10) (sic), and is arranged spaced from the inner disc (6) of the filter (5) to form the flow outlet.

21. Radiotherapy device according to one of Claims 1 to 20, characterized in that there is arranged concentrically with the housing (2) and between the radiation source (3) and the filter (5) a heat-resistant glass plate (18), whose edge is spaced with respect to the inner wall of the housing (2) by an essentially closed annular gap (1g).

22. Radiotherapy device according to one of Claims 1 to 21, characterized in that the internal diameter of the annular gap (1g) is larger than the diameter of the inner edge (22) of the flow baffle (20).

23. Radiotherapy device according to one of Claims 1 to 22, characterized in that the radiatoin source (3) is constructed as a halogen lamp with a reflector (4) pointing towards the filter (5).

24. Filter (5), which has two transparent discs (6, 7) which are fitted essentially in a plane-parallel fashion, are held in a frame (8), and delimit a closed cavity in which a medium that selectively influences the radiation spectrum is arranged, and which medium is also in contact with at least a part of the frame (8), cooling ribs (10) being provided to improve the dissipation of heat on the outer circumference of the frame (8), characterized in that the cooling ribs (10), which project outwards, reach as far as an inner wall of a housing (2), and in that at least one of the discs (6, 7) consists of a plastic disc that bends outwards like a diaphragm given a pressure rise in the cavity.

25. Filter according to Claim 24, which is further developed according to the characterizing part of one of Claims 2 ot 14.

**Revendications**

1. Dispositif d'irradiation comportant une source de rayonnement (3) disposée dans un boîtier (2) et un filtre (5) qui est disposé sur le trajet du rayonnement et qui présente deux disques transparents (6, 7) disposés sensiblement dans des plans parallèles et maintenus dans un cadre (8), qui délimitent un espace creux clos dans lequel est disposé un milieu qui influence sélectivement le spectre du rayonnement et qui est en outre en contact avec au moins une partie du cadre (8), des ailettes de refroidissement (10) étant prévues pour améliorer l'évacuation de la chaleur, caractérisé en ce que les ailettes de refroidissement (10) sont disposées à la périphérie extérieure du cadre (8) et sont conçues de façon à déborder vers l'extérieur et s'étendre jusqu'à une paroi intérieure du boîtier (2) et en ce que l'un au moins des disques (6, 7) est constitué d'un disque de plastique qui se bombe vers l'extérieur à la façon d'une membrane lorsque la pression augmente dans l'espace creux.

2. Dispositif d'irradiation selon la revendication 1, caractérisé en ce que le cadre (8) est constitué de métal ou d'un autre matériau bon conducteur de la chaleur.

3. Dispositif d'irradiation selon la revendication 1 ou 2, caractérisé en ce que le cadre (8) est conçu en deux parties, avec une partie extérieure (8a) portant les ailettes de refroidissement (10) et un cadre interchangeable (8b) qui y est maintenu de manière interchangeable et auquel les disques (6, 7) sont fixés.

4. Dispositif d'irradiation selon l'une des revendications 1 à 3, caractérisé en ce que le cadre (8) présente un épaulement périphérique d'appui qui pénètre vers l'intérieur entre les disques (6, 7) et contre lequel s'appuient les disques (6, 7).

5. Dispositif d'irradiation selon l'une des revendications 1 à 4, caractérisé en ce qu'il est prévu dans l'épaulement d'appui au moins une rainure périphérique de refroidissement (26) ouverte en direction de l'espace intérieur situé entre les disques (6, 7).

6. Dispositif d'irradiation selon l'une des revendications 1 à 5, caractérisé en ce qu'il est prévu dans l'épaulement d'appui deux rainures de refroidissement (26) l'une à côté de l'autre séparées par une nervure de refroidissement (28).

7. Dispositif d'irradiation selon l'une des revendications 1 à 6, caractérisé en ce que la rainure de refroidissement (26) est au moins aussi profonde que l'épaulement d'appui.

8. Dispositif d'irradiation selon l'une des revendications 1 à 7, caractérisé en ce que la rainure de refroidissement (26) présente un fond de rainure (27) en forme de V ou U.

9. Dispositif d'irradiation selon l'une des revendications 1 à 8, caractérisé en ce que les disques (6, 7) sont maintenus chacun par une bague de pression (29) qui appuie le disque respectif (6 ou 7) contre un côté de l'épaulement d'appui.

10. Dispositif d'irradiation selon l'une des revendications 1 à 9, caractérisé en ce que les disques (6, 7) présentent un chanfrein sur leur bord extérieur orienté vers l'épaulement d'appui, et en ce qu'une bague d'étanchéité (12) est disposée entre le chanfrein et l'épaulement d'appui.

11. Dispositif d'irradiation selon l'une des revendications 1 à 10, caractérisé en ce que le bord extérieur, opposé au disque respectif (6, 7), d'une bague de pression (29) est légèrement chanfreiné, et en ce que la face frontale extérieure du cadre (8b) entourant les bagues de pression (29) présente une rainure périphérique (31) qui laisse subsister une nervure de rabattement (32).

12. Dispositif d'irradiation selon l'une des revendications 1 à 11, caractérisé en ce que les deux disques (6, 7) sont en plastique.

13. Dispositif d'irradiation selon l'une des revendications 1 à 12, caractérisé en ce que les disques (6, 7) sont en polycarbonate.

14. Dispositif d'irradiation selon l'une des revendications 1 à 13, caractérisé en ce que des lamelles (13) rapportées sur le cadre (8) plongent dans le milieu vers l'intérieur entre les disques (6, 7).

15. Dispositif d'irradiation selon l'une des revendications 1 à 14, caractérisé en ce que le boîtier (2) est de forme sensiblement tubulaire et en ce que le cadre (8) est inséré dans le boîtier (2) par les extrémités extérieures de ses ailettes de refroidissement (10), en laissant libres des ouvertures d'écoulement de l'air.

16. Dispositif d'irradiation selon l'une des revendications 1 à 15, caractérisé en ce qu'un ventilateur (14) est disposé dans le boîtier (2).

17. Dispositif d'irradiation selon l'une des revendications 1 à 16, caractérisé en ce que le ventilateur (14) est disposé du côté de la source de rayonnement (3) opposé au filtre (5).

18. Dispositif d'irradiation selon l'une des revendications 1 à 17, caractérisé en ce qu'entre le disque (6) orienté vers la source de rayonnement (3) du filtre (5) et la source de rayonnement (3) est disposée, concentriquement au boîtier (2), une chicane annulaire d'écoulement (15, 16; 20) dont l'entrée d'écoulement est orientée vers les ailettes de refroidissement (10) du cadre (8) tandis que sa sortie d'écoulement (17) est orientée vers le filtre (5).

19. Dispositif d'irradiation selon l'une des revendications 1 à 18, caractérisé en ce que la chicane d'écoulement est conçue en demi-tore (20) dont le bord extérieur (21) jouxte le paroi intérieur du boîtier tubulaire (2) et dont le bord intérieur (22) est orienté vers le filtre (5).

20. Dispositif d'irradiation selon l'une des revendications 1 à 18, caractérisé en ce que les ailettes de refroidissement (10) se prolongent vers l'intérieur du boîtier et en ce que la chicane d'écoulement est conçue comme bague cylindrique (15) qui relie les zones de pied des ailettes de refroidissement (10), qui est fermée à l'égard du boîtier (2) par une partie en forme de flasque (10) et qui est disposée à une certaine distance du disque intérieur (6) du filtre (5) pour former la sortie de l'écoulement.

21. Dispositif d'irradiation selon l'une des revendications 1 à 20, caractérisé en ce qu'une plaquette (18) de verre résistant à la chaleur, dont le bord est écarté de la paroi intérieure du boîtier (2) par une fente annulaire (19) sensiblement continue, est disposée concentriquement avec le boîtier (2) entre la source de rayonnement (3) et le filtre (5).

22. Dispositif d'irradiation selon l'une des revendications 1 à 21, caractérisé en ce que le diamètre intérieur de la fente annulaire (19) est supérieur au diamètre du bord intérieur (22) de la chicane d'écoulement (20).

23. Dispositif d'irradiation selon l'une des revendications 1 à 22, caractérisé en ce que la source de rayonnement (3) est une lampe à halogène avec un réflecteur (4) orienté vers le filtre (5).

24. Filtre (5) présentant deux disques transparents (6, 7) disposés dans des plans sensiblement parallèles, qui sont maintenus dans un cadre (8) et délimitent un espace creux clos dans lequel est disposé un milieu qui influence sélectivement le spectre du rayonnement et qui est également en contact avec au moins une partie du cadre (8), des ailettes de refroidissement (10) étant prévues à la périphérie extérieure du cadre (8) pour améliorer l'émission de chaleur, caractérisé en ce que les ailettes de refroidissement (10) débordant vers l'extérieur s'étendent jusqu'à une paroi intérieure du boîtier (2) et en ce que l'un au moins des disques (6, 7) est constitué d'un disque de plastique qui se bombe vers l'extérieur à la façon d'une membrane lorsque la pression augmente dans l'espace creux.

25. Filtre selon la revendication 24, caractérisé en ce qu'il est perfectionné selon la partie caractérisante de l'une des revendications 2 à 14.

FIG. 1

EP 0 311 898 B1

FIG. 2

FIG. 3

FIG.4

FIG.5

FIG.6

FIG.7

FIG.8

FIG.9a

FIG.9b